# EUROPEAN PATENT APPLICATION

(11) **EP 4 582 432 A1**
(43) Date of publication of application: **09.07.2025**
(21) Application number: 23860764.2
(22) Date of filing: 23.08.2023
(51) Int. Cl.: C07F 5/00, A61K 49/10

(54) **NOVEL COMPOUND AND MRI CONTRAST AGENT COMPRISING SAME**

(30) Priority: 01.09.2022 KR 20220110725; 18.08.2023 KR 20230108317
(71) Applicant: Theranocure Co., Ltd., Daegu 41405 (KR)
(72) Inventor: CHANG, Yong Min, Daegu 41948 (KR); LEE, Sang Yun, Daegu 42400 (KR)
(74) Representative: Laine IP Oy
(86) International application number: PCT/KR2023/012456
(87) International publication number: WO 2024/049087

(57) **Abstract**

Disclosed in the present invention are a novel compound based on pyclen and an MRI contrast agent comprising same. The novel compound and MRI contrast agent of the present invention are characterized by having a structure represented by chemical formula 1-1, in which any one selected from alicyclic compounds or aromatic compounds is introduced to pyclen which is the backbone, and exhibit a stable cyclic structure. In addition, the present invention has the effect of providing a high self-relaxation rate and liver targetability.

## Description

### FIELD

The present disclosure relates to a novel compound and an MRI contrast agent comprising the same, and more specifically, to a novel compound having a high relaxivity and capable of effectively targeting a liver even in a small amount, and an MRI contrast agent comprising the same.

### DESCRIPTION OF RELATED ART

A magnetic resonance imaging (MRI) device evaluated as a general diagnostic medical device in imaging diagnosis uses electromagnetic waves and uses moisture from the human body to form an image and thus is in the spotlight as a non-invasive diagnostic device unlike X-ray. An MRI contrast agent is used together to allow the tissues or blood vessels in the body to become visible during the MRI test or procedure. When the MR contrast agent is used, a time taken to obtain the image can be shortened via rapid relaxation of water hydrogen, and tissues are well contrasted in the images such that the accuracy of diagnosis can be increased.

Recently, a gadolinium (Gd)-based contrast agent (GBCA) has been widely used. The gadolinium-based contrast agent is a compound having a structure in which a chelate ligand surrounds and binds to a gadolinium element, such as DO3A (1,4,7,10-tetraazacyclododecane 1,4,7-triacetic acid), PCTA (3,6,9,15-tetraazabicyclo 9.3. 1pentadeca-1(15), 11,13-triene-3,6,9-triacetic acid), etc. and is classified into a linear contrast agent (linear-GBCA) and a cyclic contrast agent (ocyclic-GBCA) based on the structure of the ligand. The linear contrast agent has stability problem. Thus, the cyclic contrast agent is preferred.

### DISCLOSURE

### TECHNICAL PURPOSE

One purpose of the present disclosure is to provide a novel compound based on a stable cyclic pyclen and an MRI contrast agent comprising the same.

Another purpose of the present disclosure is to provide a compound having a high targeting ability to the liver and an MRI contrast agent comprising the same.

### TECHNICAL SOLUTION

The DO3A-based gadolinium contrast agent which is mainly used in the current market is used as an extracellular agent. In particular, each of most of contrast agents used as liver contrast agents has a linear structure with low stability. Accordingly, the present disclosure provides a stable cyclic pyclen-based and liver-targeting compound.

A novel compound to achieve one purpose of the present disclosure is represented by a following Chemical Formula 1. Specifically, the compound has a structure in which a back bone is made of pyclen, and an alicyclic compound or an aromatic cyclic compound is introduced thereto.

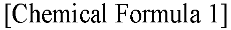

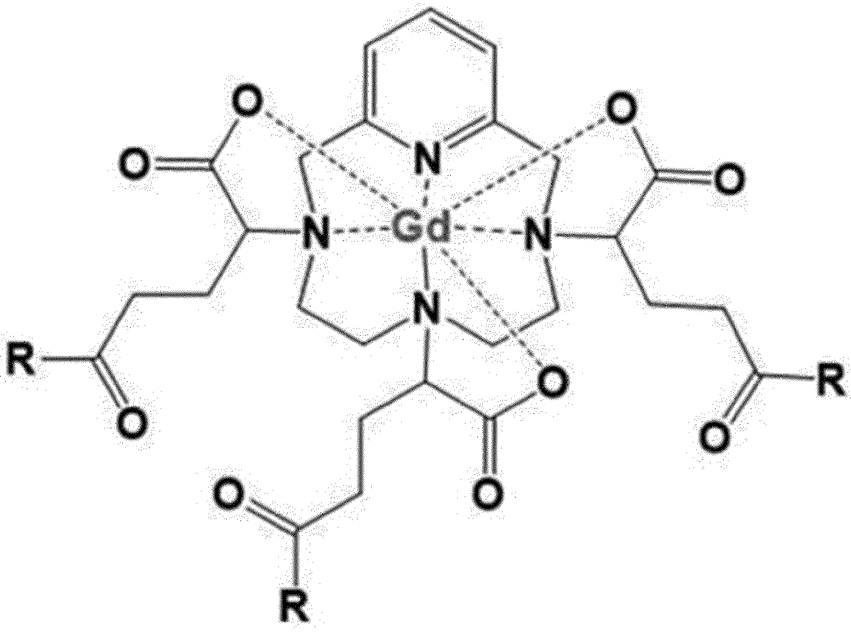

In the Chemical Formula 1, R may be one selected from an alicyclic compound and an aromatic cyclic compound.

In an embodiment, in the compound represented by the Chemical Formula 1, R may be the alicyclic compound, and the alicyclic compound may be one selected from cyclohexane and cyclopentane.

Preferably, the compound of the present disclosure may be a compound represented by a following Chemical Formula 1-1:

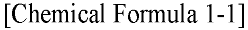

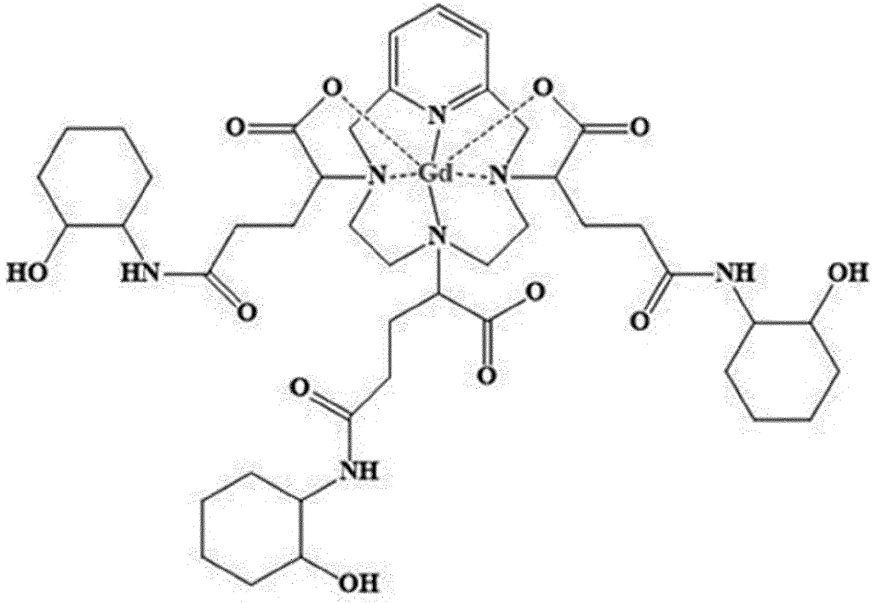

The compound represented by the Chemical Formula 1-1 may have a structure in which 2-aminocyclohexanol having a cyclic structure is introduced into a compound based on pyclen to which gadolinium binds. The compound is determined to have a liver-specific targeting effect due to lipophilicity of cyclohexane present in the 2-aminocyclohexanol. 2-aminocyclohexanol has a Log P value of 0.45, and thus exhibits the lipophilicity.

In an embodiment, the compound may specifically target the liver. Unexpectedly, when the compound as a contrast agent was injected into a blood vessel, the compound was introduced into liver cells and exhibited contrast enhancement for a long time, and also exhibited a strong contrast enhancement effect on the bile duct. In other words, the compound exhibited specificity to a hepatobiliary system.

The MRI contrast agent for achieving another purpose of the present disclosure contains the compound represented by the Chemical Formula 1. The MRI contrast agent of the present disclosure is a T₁ contrast agent and exhibits excellent contrast around a tumor or lesion. Thus, it is easy to diagnose the tumor or lesion.

In one embodiment, the MRI contrast agent may be used for diagnosis of liver metastasis of cancer, liver cyst, liver cancer, or biliary atresia.

### TECHNICAL EFFECT

According to the present disclosure, the compound has a high relaxivity to enable an image having improved quality to be provided. Further, when the compound is included in the contrast agent used as a T₁ MRI contrast agent, the T₁ MRI contrast agent can target the liver even at a small amount.

### BRIEF DESCRIPTION OF DRAWINGS

FIG. 1 is a diagram for illustrating a novel compound according to an embodiment of the present disclosure, a method for preparing the same, and an MRI contrast agent comprising the same.
FIG. 2 is a diagram for comparing and evaluating the kinetic stability of the compound Gd-PCTA-Ach according to an embodiment of the present disclosure with the kinetic stability of each of the commercial contrast agents (Gd-DOTA, Gd-HP-DO3A, Gd-DTPA, Gd-EOB-DTPA, Gd-BOPTA, and Gd-DO3A-butrol).
FIG. 3 is a diagram for comparing and evaluating the pH stability of the compound Gd-PCTA-Ach according to an embodiment of the present disclosure with that of the commercial contrast agent Gd-DO3A-butrol. FIG. 3A shows the pH stability of 0.5mM Gd-PCTA-Ach and FIG. 3B shows the pH stability of 1mM Gd-DO3A-butrol.
FIG. 4 shows the results of an experiment on the presence or absence of free Gd³⁺ in the compound Gd-PCTA-Ach according to an embodiment of the present disclosure.
FIG. 5 is a diagram for evaluating absorbance and fluorescence of each of the compounds 1mM Gd-PCTA-Ach and 0.5mM Gd-PCTA-Ach according to an embodiment of the present disclosure.
FIG. 6 is a diagram showing (A) a viability of liver cells (AML-12), (B) a viability of brain cells (C8-D1A), and (C) a viability of kidney cells (Hek-293) in mice at various concentrations of each of Gd-PCTA-Ach according to an embodiment of the present disclosure and Gd-DO3A-butrol.
FIG. 7 shows a content of Gd in each of organs when Gd-PCTA-Ach according to an embodiment of the present disclosure is injected intravenously into a normal mouse.
FIG. 8A shows an MR image based on each of Gd-PCTA-Ach and Gd-DO3A-butrol injection contents into a brain cancer model, and FIG. 8B shows a CNR graph according to the MR image.
FIG. 9A shows an MR image at 3.0T on each of Gd-PCTA-Ach according to an embodiment of the present disclosure and the commercial contrast agents Gd-BOPTA and GD-EOB-DTPA introduced into the liver in normal mice for comparison of Gd-PCTA-Ach according to an embodiment of the present disclosure with the commercial contrast agents Gd-BOPTA and GD-EOB-DTPA, and FIG. 9B shows a CNR graph according to the MR image.
FIG. 10A shows an image of normal mice in 3.0-T using Gd-PCTA-Ach according to an embodiment of the present disclosure, and FIG. 10B shows an image of normal mice in 9.4-T using Gd-PCTA-Ach according to an embodiment of the present disclosure. Yellow arrows indicate the liver and gallbladder. Data is an average ± SD, n = 3.
FIG. 11A shows (a,c) 3.0-T MRI result on the liver cancer mice using Gd-PCTA-Ach according to an embodiment of the present disclosure, and FIG. 11B shows a CNR profile for analyzing the MRI result.

### DETAILED DESCRIPTIONS

Hereinafter, embodiments of the present disclosure will be described in detail with reference to the accompanying drawings. The present disclosure may be subjected to various changes and may have various forms. Thus, particular embodiments will be illustrated in the drawings and will be described in detail herein. However, this is not intended to limit the present disclosure to a specific disclosed form. It should be understood that the present disclosure includes all modifications, equivalents, and replacements included in the spirit and technical scope of the present disclosure. While describing the drawings, similar reference numerals are used for similar components.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "including", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, a novel compound of the present disclosure and an MRI contrast agent comprising the same will be described in more detail through specific Examples and Experimental Examples. However, the Examples of the present disclosure are only some embodiments of the present disclosure, and the scope of the present disclosure is not limited to the following Examples.

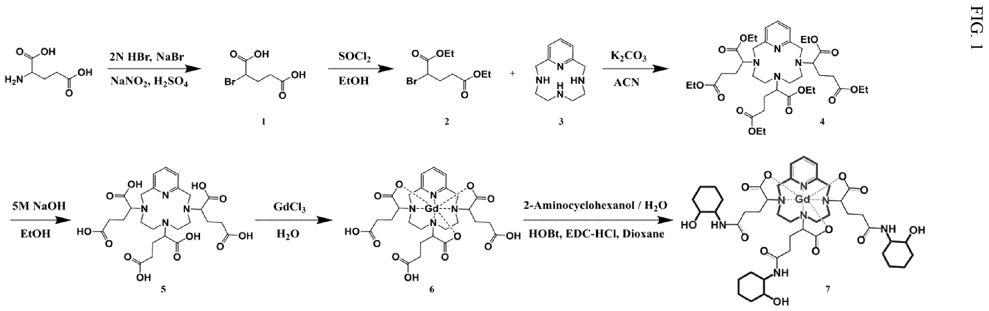

FIG. 1 is a diagram for illustrating a novel compound according to an embodiment of the present disclosure, a method for preparing the same, and an MRI contrast agent comprising the same. Hereinafter, a novel compound of the present disclosure and an embodiment thereof will be described with reference to FIG. 1.

### Examples

### Compound 1 (2-bromopentanedioic acid)

L-glutamic acid (10 g, 67.9mmol) and NaBr (24.40g, 237mmol) were dissolved in a 2N HBr solution. NaNO₂ (8.485g, 123mmol) was slowly added to the solution in a small amount in a solid form over 1 hour at 0°C. After 2 hours and 30 minutes, concentrated sulfuric acid was slowly added dropwise to a yellow mixture at room temperature, and then the orange-brown solution was stirred for 20 minutes. The solution was subjected to extraction with diethyl ether (4 x 100 mL), and the thus collected organic layer was extracted with brine (2 x 100 mL) and dried over Na₂SO₄. Subsequently, the solvent was removed therefrom under reduced pressure, and thus, Compound 1 was obtained without further purification. Yield: 36%.

### Compound 2 (Diethyl-2-bromopanedioate)

The solution (5.14g, 24.48mmol) of the compound 1 as prepared above was dissolved in EtOH (200mL, excess). Thionyl chloride (3.55 mL) was slowly added dropwise to the solution at 0°C. The pale-yellow solution was stirred at room temperature for 2 days. The solution was subjected to evaporation and the resulting oil was dissolved in DCM (100 mL). The mixture was subjected to extraction with 5% NaHCO₃ (4× 100mL) and the thus collected organic layer was extracted with brine (2× 100mL) and dehydrated with Na₂SO₄. The solvent was removed therefrom under reduced pressure. The thus obtained product was then purified by column chromatography using petroleum ether and ethyl acetate to obtain Compound 2. Yield: 49.2%.

### Compound 3 (3,6,9,15-Tetraazabicyclo9,3,1pentadeca-1(15),11,13- triene)

Compound 3 was supplied from ACG-PHARM and was used.

### Compound 4 (Hexaethyl 2,2',2"-(3,6,9-triaza-1(2,6)-pyridinacyclodecaphane-3,6,9-triyl)trigluatrate)

A solution of the compound 2 (5.98g, 22.47mmol) of ACN (acetonitrile) and K₂CO₃ (4.12g, 29.88mmol) were added to a solution of the compound 3 (0.77g, 3.735mmol) of ACN, and the resulting mixed solution was refluxed at 65°C for 24 hours. K₂CO₃ was then removed therefrom with a paper filter. The filtered solvent was blown off with an evaporator, and then, the concentrated solution was added to ethyl acetate (100 mL), followed by extraction with 0.1M HCl (4 x 100mL). The thus collected aqueous layer was added to 5% NaHCO₃ to adjust the pH thereof to 7 to 8 in an ice bath. The solution was then subjected to extraction with DCM (3 x 400mL) and the thus obtained organic layer was dried using Na₂SO₄. Then, the solvent was evaporated away, and thus, Compound 4 was obtained without further purification. Yield: 65%

### Compound 5 (2,2',2"-(3,6,9-triaza-1(2,6)-pyridinacyclodecaphane-3,6,9-triyl)triglutaric acid)

Compound 4 as prepared above was dissolved in EtOH to prepare a solution which in turn was added to 5M NaOH. Then, the mixed solution was refluxed at 80°C for 23 hours. The solvent was evaporated away, and the pH of the mixed solution was adjusted to 7 by using HCl (conc HCL) at 0°C. The resulting product was then purified by ion chromatography, elution water, and ACN without a buffering condition. Then, the purification product was subjected to filtering using a syringe filter, and then, the solvent was blown away and the resultant was lyophilized, and thus, a yellow compound 5 was obtained without further purification. Yield: 69%

### Compound 6 (GdL)

Compound 5 (0.51g, 0.855mmol) as obtained above was dissolved in HPLC water and PH of the resulting solution was adjusted to pH 7 with 1M NaOH. A solution in which GdCl₃ was dissolved in water was then slowly added to the above prepared solution. pH of the solution was then adjusted to pH 7 with 1M NaOH and the solution was stirred at room temperature for 24 hours. A dark yellow solution was then filtered through paper. The resulting product was purified by prep HPLC using a hydrosphere C18 column, elution (flow rate: 8 mL/min) neutral water, neutral ACN conditions as follows:
0 min 5% solvent B, 3 min 40% solvent B, 28 min 80% solvent B, 31 min 100% solvent B, 34 min 100% solvent B, 37 min 5% solvent B.

Then, the purification product was subjected to filtering using a syringe filter, and then, the solvent was evaporated away therefrom, and the resultant was lyophilized to obtain Compound 6 (GdL) of the present disclosure.

### Compound 7 (Gd-PCTA-Ach)

Compound 6 (1g, 1.331mmol) as obtained above was dissolved in HPLC water. A solution in which HOBt (0.359g, 2.662mmol) was dissolved in dioxane and a solution win which EDC·HCl (1.27g, 6.655mmol) was dissolved in water were sequentially slowly added dropwise to the above prepared solution, followed by stirring for 10 minutes. A solution in which 2-aminocyclohexanol (0.91g, 7.986mmol) was dissolved in water was slowly added to the above prepared solution, and then, pH of the resultant solution was adjusted to pH 6 using 1M HCl, followed by stirring at room temperature for 24 hours. Then, the mixed solution was filtered through paper. The obtained product was purified by Prep HPLC using a Triart C18 column during following two steps:

First, elution (flow rate: 12 mL/min) water, MeOH conditions are as follows: 0 min 0% solvent B, 5 min 0% solvent B, 60 min 90% solvent B, 70 min 90% solvent B, 75 min 0% solvent B. Second, elution (flow rate: 12 mL/min), ACN conditions are as follows: 0 min 0% solvent B, 5 min 0% solvent B, 60 min 90% solvent B, 70 min 90% solvent B, 75 min 0% solvent B.

Then, the purification product was subjected to filtering using a syringe filter, and then, the solvent was evaporated away therefrom, and the resultant was freeze-dried to obtain the compound Gd-PCTA-Ach of the present disclosure. Yield: 10%

### Comparative Example

In order to compare and evaluate the performance of Gd-PCTA-Ach of the present disclosure with the performance of each of commercial contrast agents such as Dotarem^{®} (Gd-DOTA), Prohance^{®} (Gd-HP-DO3A), Magnevist^{®} (Gd-DTPA), Primovist^{®} (Gd-EOB-DTPA), Multihance^{®} (Gd-BOPTA), Gadovist^{®} (Gd-DO3A-butrol) and Gadopiclenol, the commercial contrast agents were used as Comparative Examples.

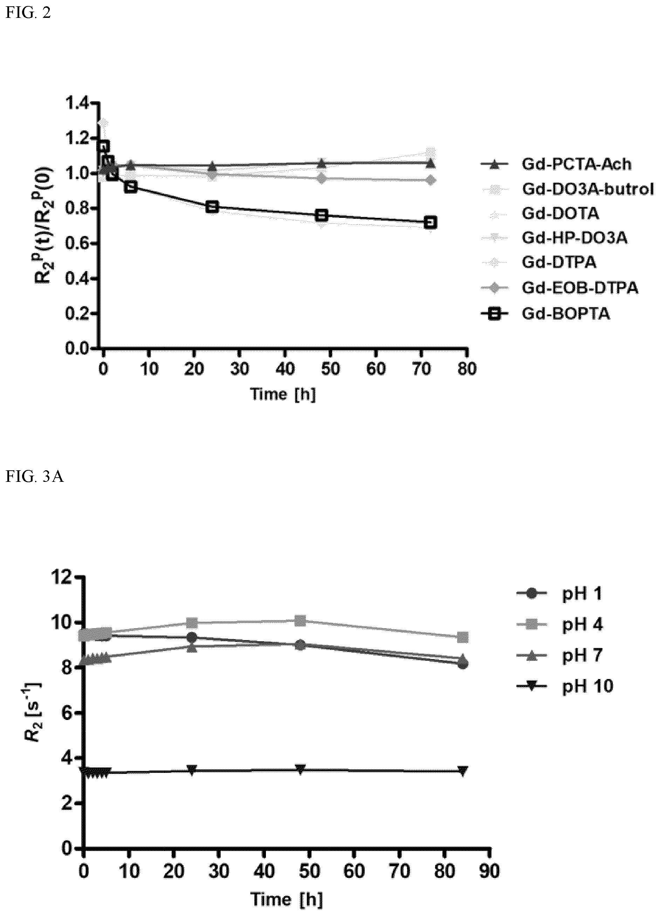

FIGS. 2 to 11 are diagrams showing results according to respective Experimental Examples of the present disclosure. Hereinafter, the characteristics of the novel compound of the present disclosure and the effect of the MRI contrast agent including the same will be described with reference to FIGS. 2 to 11.

### Experimental Example

### Experimental Example 1: Evaluation of Relaxability and HSA Binding

Relaxability (r₁ and r₂), hydrophilicity (Log P) and HSA binding affinities of the Compound Gd-PCTA-Ach of the present disclosure and the commercial contrast agents were compared with each other and evaluated. The relaxability (r₁ and r₂) was measured under 3.0T and 9.4T magnetic fields at water and HSA, respectively. The HSA binding affinity was measured using ultrafiltration analysis. The measurement result values are summarized in Table 1 as set forth below.

**Table 1**

| | 3.0-T | | | | 9.4-T | | log P (log Cₒ/C_{w}) | HSA bindin g affinity (%) |
|---|---|---|---|---|---|---|---|---|
| | Water | | HSA | | Water | | | |
| | r₁ (mM⁻¹s⁻¹) | r₂ (mM⁻¹s⁻¹) | r₁ (mM⁻¹s⁻¹) | r₂ (mM⁻¹s⁻¹) | r₁ (mM⁻¹s⁻¹) | r₂ (mM⁻¹s⁻¹) | | |
| Gd-PCTA-Ach | 16.3±0.1 | 21.0±0.1 | 14.9±0.1 | 21.4±0.2 | 10.3±1.9 | 18.5±3.3 | -1.0 | 34.8 |
| Gd-DO3A-butrol | 4.3±0.2 | 5.5±0.02 | 4.3±0.1 | 6.5±0.1 | 4.9±0.9 | 6.4±1.1 | -3.1 | 3.4 |
| Gd-BOPTA | 5.1±0.02 | 6.8±0.4 | 5.8±0.2 | 9.0±0.3 | 6.1±1.1 | 8.1±1.4 | -2.9 | 15.0 |
| Gd-EOB-DTPA | 6.5±0.04 | 7.3±0.4 | 7.7±0.1 | 11.1±0.04 | 7.4±1.4 | 10.7±1.9 | -2.9 | 8.9 |
| Gadopiclenol^{a} | 11.3 | 13.5 | | | | | -4.2 | |

Referring to Table 1, the Gd-PCTA-Ach of the present disclosure exhibited higher relaxability (r1 and r2) than that of each of the commercial contrast agents under all conditions.

The Log P value is a value for evaluating the hydrophilicity. When the P value is smaller than 1, the Log P value becomes negative to indicate the hydrophilicity. When the P value is greater than 1, the Log P value becomes positive to indicate lipophilicity. The Gd-PCTA-Ach of the present disclosure was identified to have the hydrophilicity because the Log P value thereof was -1.0 and thus is negative.

Further, a binding percentage value of Gd-PCTA-Ach was identified to be threefold higher than that of each of Gd BOPTA and Gd-EOB-DTPA. On the basis of the hydrophilic result, the log P value causes a strong interaction with blood proteins such as HSA, and the HSA binding assay data support this fact. As a result, it may be determined that the Gd-PCTA-Ach will stay in the blood for a long time based on the high HAS binding value of 30. In the HSA binding assay, Gd-PCTA-Ach may have a long half-life for which the Gd-PCTA-Ach will stay in the blood. However, it is necessary to investigate actual accumulation thereof in hepatocytes in vivo, regardless of whether there is a greater interaction thereof with HSA or whether it carries proteins from hepatocytes. Additional data on accumulation thereof in the hepatocyte were demonstrated via in vivo MRI experiments.

### Experimental Example 2: Stability Evaluation

### (1) Evaluation of Dynamic Stability

The dynamic stability experiment is an experiment to find out how much Gd³⁺ binding to the ligand is exchanged with zinc abundantly distributed in the human body. The free Gd³⁺(transmetalation) replacing the zinc may be harmful to the human body because it is toxic. That's why it's important not to exchange therebetween for a long time. In the dynamic stability experiment, the commercial contrast agents (Gd-DOTA, Gd-HP-DO3A, Gd-DTPA, Gd-EOB-DTPA, Gd-BOPTA, and Gd-DO3A-butrol) were compared with two concentrations of Gd-PCTA-Ach (1.5mM, 1mM), and the results are shown in FIG. 2.

Referring to FIG. 2, the metal exchange amount between Gd³⁺ and zinc in the drug over time is shown. Based on the observation result for 72 hours, the metal exchange amount of Gd-PCTA-Ach was similar to values of Gd-DOTA, Gd-HP-DO3A, and Gd-DO3A-butrol, and was higher than the values of Gd-DTPA, Gd-EOB-DTPA, and Gd-BOPTA.

### (2) pH Stability Assessment

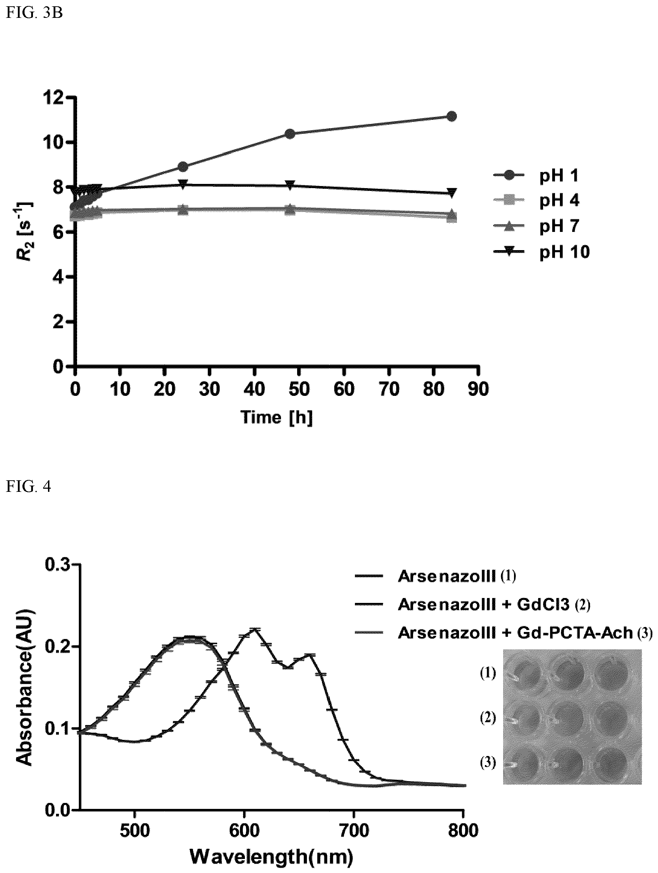

The pH stability test is an experiment that identifies the stability of the drug at each of pHs. Although the pH of the human body is usually maintained at around 7.4, pHs of various portions thereof are slightly different from each other. Thus, it is also important to check the stability at each of pHs. The pH stability experiment was performed for 72h in the same manner as in the dynamic stability evaluation, and the stability was identified under the conditions of pH 1, 4, 7, and 10, respectively. The results are shown in FIG. 3.

Referring to FIG. 3, it may be identified that pH is constantly maintained for 72 hours. Although the value varies according to varying pH, the stability was observed. The Gd-PCTA-Ach of the present disclosure exhibited high stability as the pH increased. When the pH was 7 or higher, the stability thereof was lower than that of Gd-DOTA as a commercial contrast agent.

### Experimental Example 3: Evaluation of Other Chemical Properties

### (1) Evaluation of Presence or Absence of Free Gd³⁺

Since the free Gd³⁺ is toxic, an experiment was performed to determine whether the free Gd³⁺ is present in the synthesized Gd-PCTA-Ach. The presence of free Gd³⁺ was identified by graphing the absorbance using Arsenazo III as a dye, and the result is shown in FIG. 4.

Referring to FIG. 4, it is identified that the Gd-PCTA-Ach of the present disclosure does not have the free Gd³⁺ because it appears purple and red forms unlike Arsenazo III and free Gd³⁺.

### (2) Evaluation of Light Absorption and Fluorescence

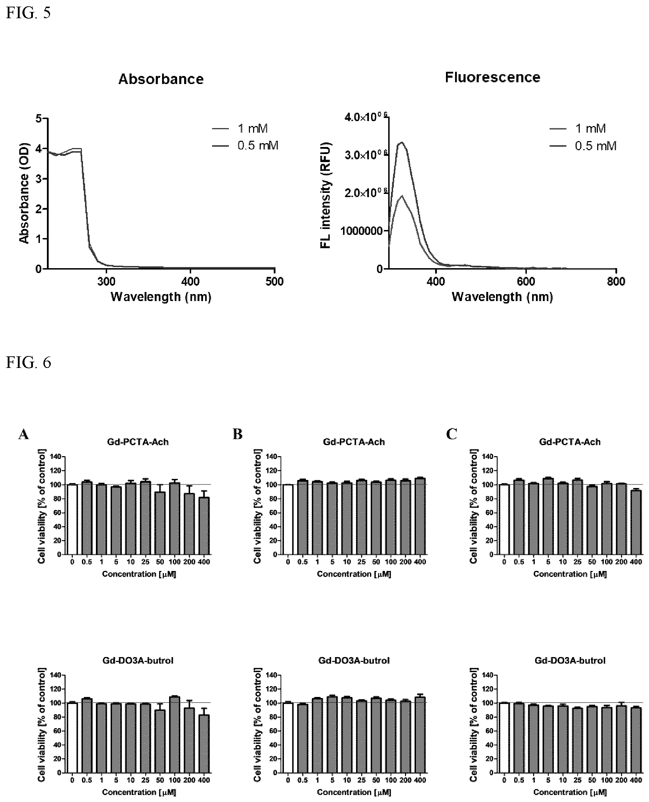

An experiment was performed to determine the absorption wavelength and emission wavelength of the Gd-PCTA-Ach synthesized through the present disclosure. In this experiment, the concentration of Gd-PCTA-Ach was 1 mM and 0.5mM, respectively, and the result is shown in FIG. 5.

Referring to FIG. 5, the absorption wavelength was highest at 270nm, and the emission wavelength was highest at 315nm.

### Experimental Example 4: Cytotoxicity

FIG. 6 shows the results of cytotoxicity experiments of Gd-PCTA-Ach and Gd-DO3A-butrol in mouse hepatocytes (A), brain cells (C8-D1A), and kidney cells (Hek-293), respectively. Gd-DO3A-butrol was used as a positive group, and cells were incubated with the drug for 24 hours.

Referring to FIG. 6, it is identified that when Gd-PCTA-Ach of the present disclosure and Gd-DO3A-butrol are compared with each other, Gd-PCTA-Ach of the present disclosure is not cytotoxic like Gd-DO3A-butrol used in the market.

### Experimental Example 5: Evaluation of Biodistribution

In order to confirm the biodistribution, normal mice were injected intravenously with the compound of the present disclosure (Gd-PCTA-Ach), and after 15 minutes, 30 minutes, 2 hours and 24 hours, the organ in which the compound was detected was identified via ICP.

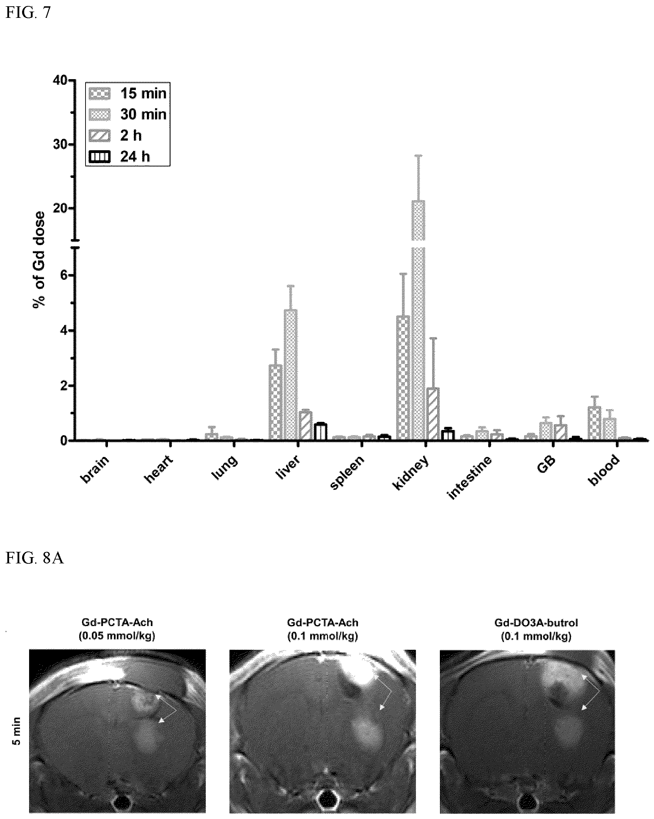

FIG. 7 shows the content of Gd per organ. It was identified that Gd-PCTA-Ach was introduced into the liver as the intensity was measured to be high in the liver and kidneys among organs. In addition, the compound was also detected in gallbladder (GB) and blood vessels (Blood). According to these results, the compound of the present disclosure has characteristics similar to that of Gd-BOPTA which is a commercial contrast agent used as a hepatobiliary specific contrast agent.

### Experimental Example 6: Image Evaluation

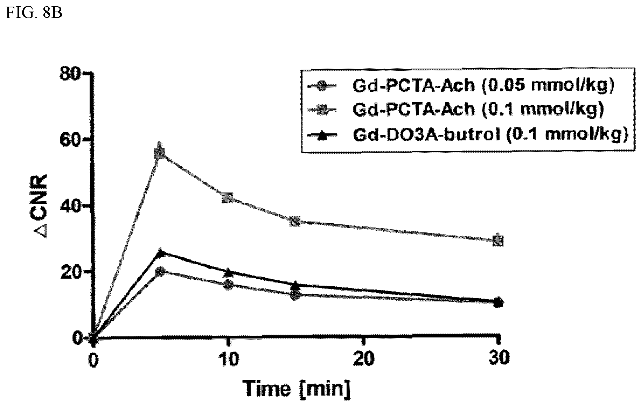

After a brain cancer model was prepared using C6 glioma which is a brain cancer cell, 0.1mmol/kg of Gd-PCTA-Ach 0.1mmol/kg, Gd-PCTA-Ach 0.05mmol/kg, and Gd-DO3A-butrol were injected thereto, respectively, to obtain MR images (FIG. 8A) and CNR graphs (FIG. 8B) according to the MR images, and the results are shown in FIG. 8.

Referring to FIG. 8A, it was observed that the brain cancer site appeared brighter in the 0.1mmol/kg of the Gd-PCTA-Ach of the present disclosure than in the 0.1 mmol/kg of Gd-DO3A-butrol. In addition, 0.1 mmol/kg of Gd-DO3A-butrol exhibited a brightness comparable to that of 0.05 mmol.kg, which is a half concentration of Gd-PCTA-Ach. Referring to FIG. 8B, it may be identified that the changes in the CNR of 0.1 mmol/kg of Gd-DO3A-butrol and 0.05 mmol/kg of Gd-PCTA-Ach are substantially equal to each other. Therefore, it may be identified that when the Gd-PCTA-Ach of the present disclosure is used, an image of the similar brightness can be provided even when the Gd-PCTA-Ach of the present disclosure is used at a lower concentration than the used concentration of the commercial contrast agent Gd-DO3A-butrol.

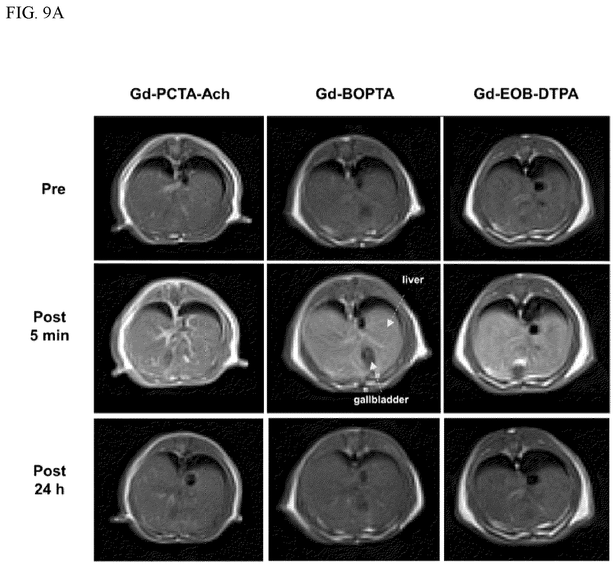

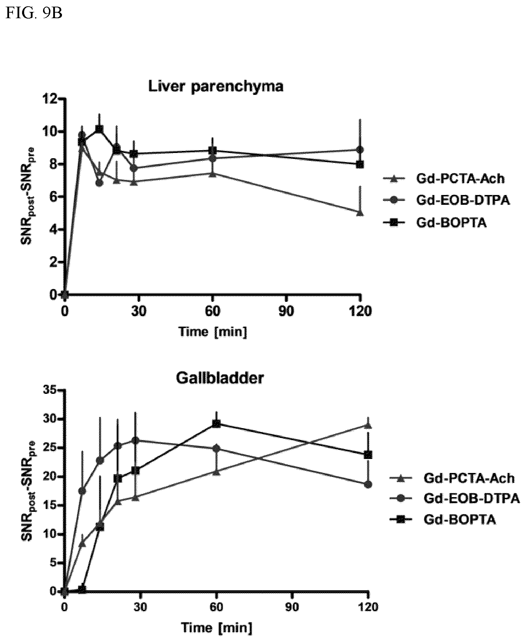

FIG. 9A shows an MR image at 3.0T on each of Gd-PCTA-Ach according to an embodiment of the present disclosure and the commercial contrast agents Gd-BOPTA and GD-EOB-DTPA introduced into the liver in normal mice for comparison of Gd-PCTA-Ach according to an embodiment of the present disclosure with the commercial contrast agents Gd-BOPTA and GD-EOB-DTPA, and FIG. 9B shows a CNR graph according to the MR image.

Referring to FIG. 9, at 5 minutes after the injection of all three MR contrast agents to the liver, the liver parenchyma was observed, and then, the observed amount of the liver parenchyma decreased slowly. On the other hand, the gallbladder was observed slightly differently. In the case of Gd-BOPTA and Gd-EOB-DTPA, CA was initially enhanced, and then was observed to decrease slowly, whereas in the case of Gd-PCTA-Ach, CA was observed to gradually increase as the time elapsed from 5min to 120min. The clearances of all three MR CA were identified at 24 hours after intravascular injection. Gd-PCTA-Ach uniquely exhibited a transient blood enhancement than other agents, and then, Gd-PCTA-Ach entered the liver at a slightly lower enhancement than Gd-BOPTA and Gd-EOB-DTPA.

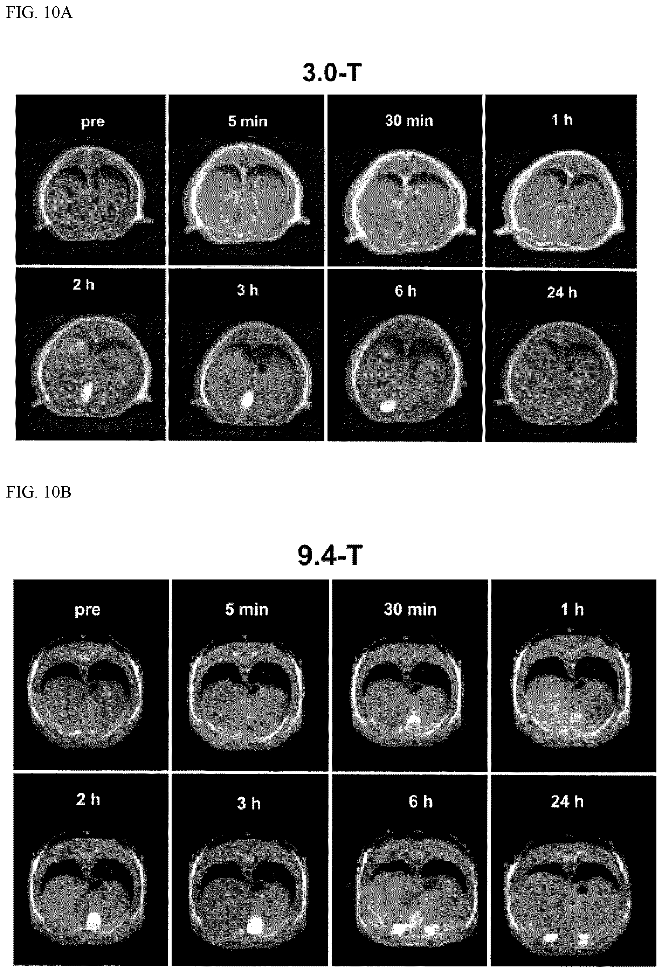

FIG. 10A shows an image of normal mice in 3.0-T using Gd-PCTA-Ach according to an embodiment of the present disclosure, and FIG. 10B shows an image of normal mice in 9.4-T using Gd-PCTA-Ach according to an embodiment of the present disclosure. Mice were injected intravenously with the Gd-PCTA-Ach according to an embodiment of the present disclosure at a dose of 0.1mmol/kg Gd-PCTA-Ach.

Referring to FIG. 10, it is generally known that the relaxability decreases as the MHz increases. Referring to the MR image pattern of Gd-PCTA-Ach of the present disclosure based on the change in the magnetic field (3.0T(64MHz)->9.4T(400MHz)), it is identified that the relaxability r₁ and r₂ of the Gd-PCTA-Ach decreases because the change in the magnetic field has increased. Therefore, regarding the improvement of the contrast, a brighter image quality is achieved at 3T than at 9.4T.

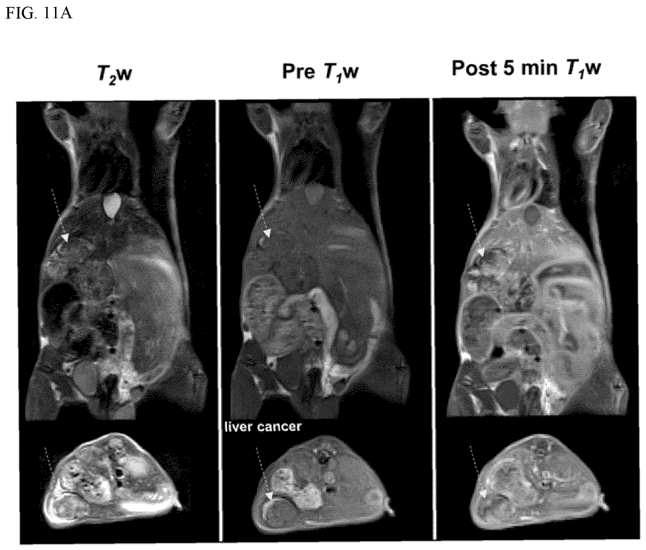

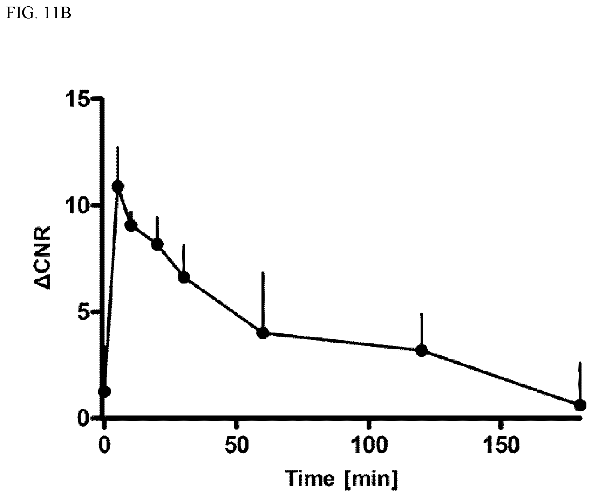

FIG. 11A shows (a,c) 3.0-T MRI result on the liver cancer mice using Gd-PCTA-Ach according to an embodiment of the present disclosure, and FIG. 11B shows a CNR profile for analyzing the MRI result. The HepG2 cell line was injected into orthotopic hepatocellular carcinoma (HCC) mice which in turn were grown until the tumor was sufficiently large. After the tumorigenesis, the HCC mice were injected intravenously with 0.1mmol/kg of Gd-PCTA-Ach.

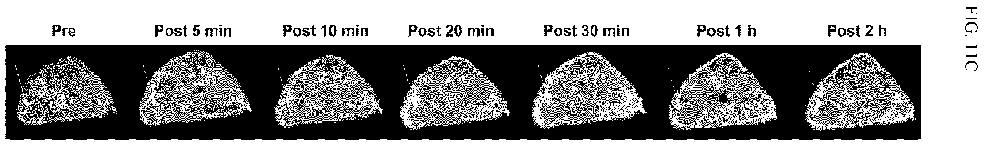

Referring to FIG. 11, T₂-weighted images were used to confirm the tumor location prior to the Gd-PCTA-Ach injection. The tumor was not clearly visible in the pre-injection T₁-weighted image (a). It was identified that in the liver cancer, the boundary between the normal group and the tumor group became clear at 5 minutes after the Gd-PCTA-Ach injection, and the image contrast of the tumor itself was slightly strengthened and then gradually decreased (A and c). HCC is known as an angiogenic tumor. It may be assumed that Gd-PCTA-Ach enters the tumor's blood vessels and exhibits intertumor contrast enhancement. As shown in (b), the contrast effect of Gd-PCTA-Ach exhibited a great improvement in 5 minutes and then gradually decreased.

Although the present disclosure has been described above with reference to the preferred embodiments of the present disclosure, those skilled in the art will understand that the present disclosure may be variously modified and changed within the scope not departing from the spirit and scope of the present disclosure described in the following patent claims.

## Claims

1. A compound represented by a following Chemical Formula 1: 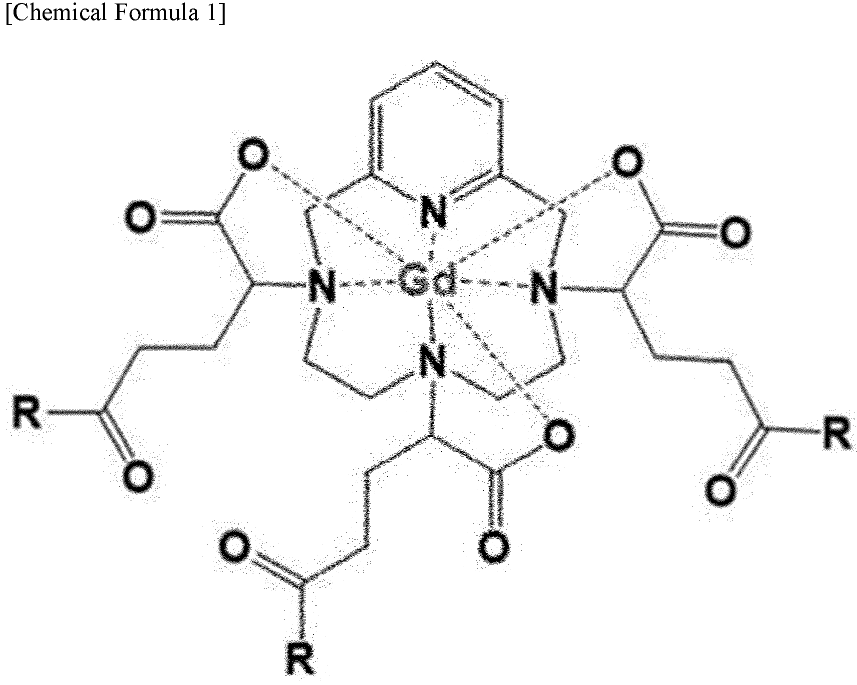 wherein in the Chemical Formula 1, R represents one selected from an alicyclic compound and an aromatic cyclic compound.

2. The compound of claim 1, wherein in the Chemical Formula 1, R is the alicyclic compound,
wherein the alicyclic compound is one selected from cyclohexane and cyclopentane.

3. The compound of claim 2, wherein the compound is represented by a following Chemical Formula 1-1:

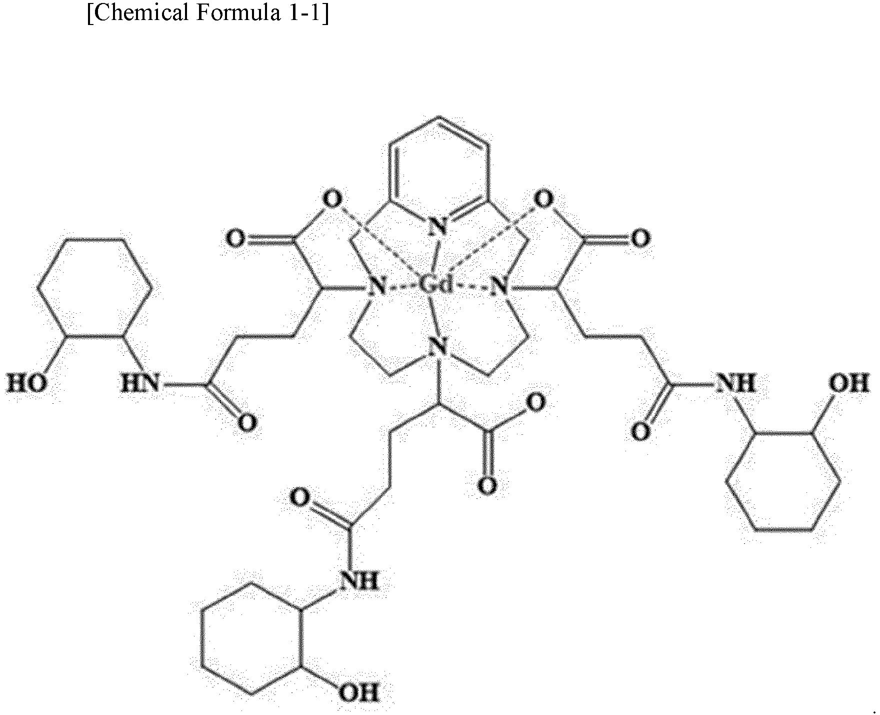

4. The compound of claim 1, wherein the compound specifically targets a liver.

5. An MRI contrast agent containing the compound according to one of claims 1 to 4.

6. The MRI contrast agent of claim 5, wherein the MRI contrast agent is used in diagnosis of liver metastasis of cancer, liver cyst, liver cancer, or biliary atresia.
